# EUROPEAN PATENT SPECIFICATION

(11) **EP 3 202 456 B1**
(45) Date of publication and mention of the grant of the patent: **31.05.2023**
(21) Application number: 15847451.0
(22) Date of filing: 24.07.2015
(51) Int. Cl.: A61N 1/05, A61N 1/36

(54) **DEEP BRAIN STIMULATION DEVICE**
VORRICHTUNG ZUR TIEFEN HIRNSTIMULATION
DISPOSITIF DE STIMULATION CÉRÉBRALE PROFONDE

(30) Priority: 30.09.2014 CN 201410517312
(43) Date of publication of application: 09.08.2017
(73) Proprietor: Sceneray Co., Ltd, Suzhou, Jiangsu 215123 (CN)
(72) Inventor: DOU, Meijuan, Suzhou Jiangsu 215123 (CN)
(74) Representative: Cabinet Beaumont
(86) International application number: PCT/CN2015/085005
(87) International publication number: WO 2016/050114

(56) References cited:
- CN-A- 104 189 995
- CN-U- 204 092 843
- US-A1- 2007 203 546
- US-A1- 2010 057 159
- US-A1- 2011 301 665
- US-A1- 2012 053 658
- US-A1- 2012 165 898
- US-A1- 2013 105 071

## Description

### BACKGROUND

### 1. TECHNICAL FIELD

The present disclosure relates to a lead, a device and a method for deep brain stimulation, which belongs to the technical field of implantable medical devices.

### 2. DESCRIPTION OF RELATED ART

The midbrain-edge-cortex system reward circuits are relevant to drug-addiction, Obsessive-Compulsive Disorder (OCD) or depression etc. For instance of drug-addiction, drug abuse and drug dependence are social problems world widely. 2014 Report for Drug Rehabilitation in China illustrated that the accumulated registered drug addicts were 2,475 millions. 1,358 millions of them were addicted to opoids, which occupied 54.9% of all.

In 2013, newly registered drug addicts were 0.365 millions. The actual amount of drug addicts is 10 times of the registered. Thus the drug addicts are much more than currently accounted. Drug rehabilitation is required.

Drug rehabilitation includes detoxification and relapse prevention. Medically assisted detoxification alone is inefficient as a treatment for addiction, because it is hard to overcome psychological dependence. The rate of drug re-abuse within six months is up to 97%, and the patients is sank into the circle of "abuse - detoxification - re-abuse". The key point to prevent re-abuse is to control the psychological dependence. Document US-A-2012/053658 discloses the most relevant prior art.

### SUMMARY

The invention is defined in claim 1. Any embodiment which is in contradiction to the subject-matter of claim 1 is not part of the invention.

The present disclosure provides a lead for deep brain stimulation including a lead body having a proximal section adapted to be electronically coupled to a power source and a distal section having at least two electrode groups. The at least two electrode groups include a first electrode group and a second electrode group. The first electrode group includes at least one first electrode which is adapted for being positioned in a nucleus accumben of a brain. The second electrode group includes at least one second electrode which is adapted for being positioned in an anterior limb of an internal capsule of the brain. The first electrode group and the second electrode group work together to stimulate targets in the brain. In one embodiment of the present disclosure, the first electrode group and the second electrode group simultaneously stimulate the targets in the brain.

The present disclosure provides a device having the lead described above. The device further includes a control module outside of a brain, a power module electrically connected with control module and a stimulation output module electrically connected with the control module and the power module.

The present disclosure provides a method for deep brain stimulation including the steps of: S1: implanting the lead into a brain of a patient; and S2: activating the lead to make the first electrode group and the second electrode group working together to stimulate the brain.

The foregoing has outlined rather broadly the features and technical advantages of the present disclosure in order that the detailed description of the invention that follows may be better understood. Additional features and advantages of the invention will be described hereinafter which form the subject of the claims of the invention.

### BRIEF DESCRIPTION OF THE DRAWINGS

The components in the drawing are not necessarily drawn to scale, the emphasis instead being placed upon clearly illustrating the principles of the described embodiments. In the drawings, reference numerals designate corresponding parts throughout various views, and all the views are schematic.
FIG. 1 is a structural schematic view of a DBS device which has been positioned in a human brain;
FIG. 2 is a structural schematic view of DBS lead positioned in the brain in accordance with an embodiment of the present disclosure;
FIG. 3 is a structural schematic view of the DBS lead in accordance with an embodiment of the present disclosure;
FIG. 4 is a structural schematic view of electrodes and contacts of the DBS lead in accordance with another embodiment of the present disclosure;
FIG. 5 is a structural schematic view of the DBS device in accordance with an embodiment of the present disclosure; and
FIG. 6 is a schematic view showing a method for DBS in accordance with an embodiment of the present disclosure.

### DETAILED DESCRIPTION OF ILLUSTRATED EMBODIMENTS

Reference will now be made to the drawing figures to describe the embodiments of the present disclosure in detail. In the following description, the same drawing reference numerals are used for the same elements in different drawings.

The present disclosure refers to a DBS lead for activating directed electrical stimulation to functional areas in a brain. The said functional areas include nerve nucleuses or nervous tracts with specific functions, which include but not limited to nucleus accumbens and an anterior limb of an internal capsule etc. The present disclosure can be applied for the therapy of psychological disorders, such as drug-addiction, OCD and depression etc. It is also therapeutic benefit for disorders sensitive to the DBS. The present disclosure illustrates a therapy of drug-addiction. However, it is understandable that the present application is not limited to this illustration.For better understanding, in the present disclosure, positions and directions are defined by the reference of a pulse generator. For example, an end close to the pulse generator is defined as a proximal section, and an end far from the pulse generator is defined as a distal section.

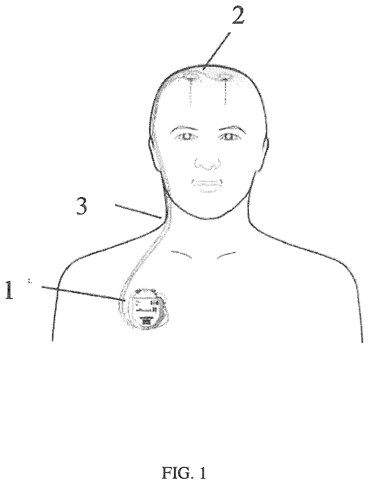

FIG. 1 schematically illustrates an implantable DBS device positioned in a brain. The device includes a pulse generator 1, two leads 2 and an external control device (not shown). The pulse generator 1 is connected with the leads 2 via conductive wires 3 so that the pulse generated by the pulse generator 1 can be transmitted to the leads 2. The pulse signal is transferred from contacts to specific neuron targets in the brain for stimulating the specific neuron targets so as to refresh the function of the human body. Besides, the external control device includes a doctor monitor and a patient controller.

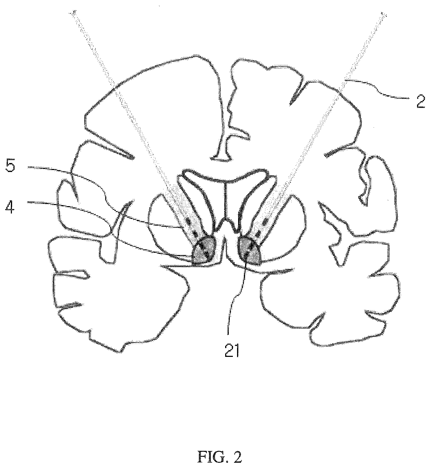

FIG. 2 schematically depicts two stimulation leads 2 deeply positioned in the brain. FIG. 2 schematically depicts the leads 2 which are partially embedded simultaneously in the nucleus accumbens 4 and the anterior limbs of the internal capsules 5 at the left side and the right side of the brain. In the invention, it is proved that the nucleus accumbens 4 and the anterior limbs of the internal capsules 5 are the two most active stimulation targets in the therapy of drug-addiction. Each nucleus accumben 4 is located adjacent to corresponding anterior limb of the internal capsule 5.

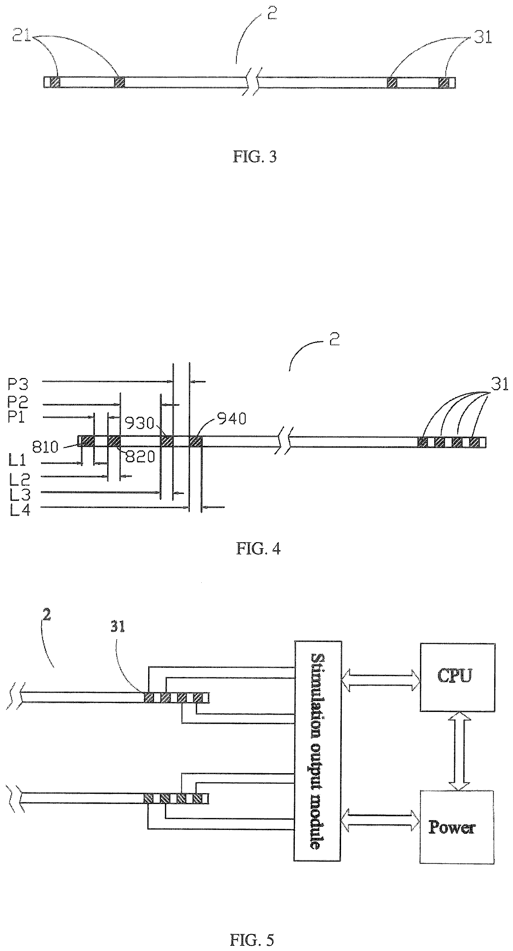

FIG. 3 schematically discloses a kind of lead 2 in an embodiment. The lead 2 includes at least two electrode groups 21 in the distal section and at least two contact groups 31 in the proximal section. The two electrode groups 21 include a first electrode and a second electrode. Similarly, the two contact groups 31 include a first contact and a second contact. Concretely, the lead 2 is of a cylinder configuration. Either the electrodes 21 or the contacts 31 is of an annular configuration which surrounds the circumference surface of the lead 2. The electrodes and the contacts are electrically connected with each other, for example via conductive lines 3. The amount of the contacts is equal to that of the electrodes, and each electrode is connected to a corresponding contact. The first and the second electrodes are implanted into the target areas of the brain for accurate stimulation. The first and the second contacts are connected to a control module for receiving and transmitting stimulation signals to the first and the second electrodes.

In order to accurately cover these two functional areas of the nucleus accumben 4 and the anterior limb of the internal capsule 5, the electrodes need to be well designed so as to adapt the structures of the nucleus accumben 4 and the anterior limb of the internal capsule 5.

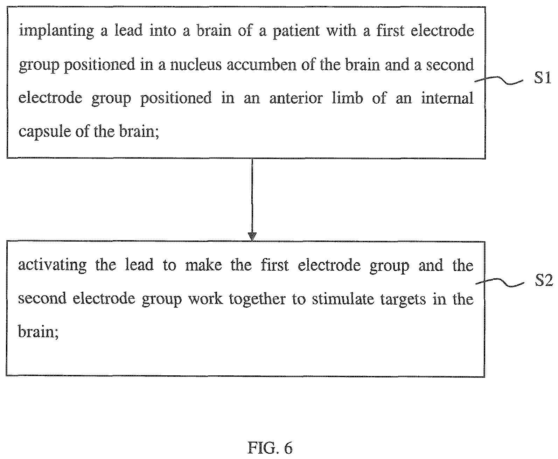

FIG. 2 and FIG. 6 depict that the first electrode is positioned in the nucleus accumben 4 and the second electrode is positioned in the anterior limb of the internal capsule 5 by surgery. As a result, the two functional areas of the brain are covered by a single lead 2 therein. Latterly, continual electrical stimulation will be applied to jointly stimulate the addicted cells at these two functional areas so as to resist a pathological drug-adhesive nerve center. Each single electrode can be adjusted by the control module via corresponding contact. In accordance with the illustrated embodiment of the present disclosure, the control module delivers distinctive stimulation parameters, i.e., pulse amplitude, pulse width or pulse frequency etc., to the nucleus accumben 4 and the anterior limb of the internal capsule 5.

Referring to FIG. 4, in another embodiment of the present disclosure, the electrode group 21 include two first electrode 810, 820 disposed along an axis direction of the lead 2 and two second electrode 930, 940 disposed along the axis direction. Stimulation frequency of the first electrodes 810, 820 is lower than that of the second electrodes 930, 940. In other words, the pulse frequency applied to the anterior limb of the internal capsule 5 is higher than that to the nucleus accumben 4. Besides, the stimulation parameters can be optimized via continual adaptation.

The first distance between the two first electrodes 810, 820 is P1. The second distance between the two electrode groups 21 is P2. The second distance P2 is larger than the first distance P1. As a result, the first electrodes 810, 820 act on the nucleus accumbens 4, and the second electrodes 930, 940 act on the anterior limbs of the internal capsule 5. Alternatively, the first electrodes 810, 820 can act on a first functional area in the brain, while the second electrodes 930, 940 can act on a second functional area adjacent to the first functional area.

In accordance with an illustrated embodiment of the present disclosure, a third distance between the second electrodes 930, 940 is P3. The third distance P3 is no less than 0.5mm. It is understandable that the third distance P3 can be elongated to cover more areas so as to adapt the anterior limb of the internal capsule 5 having large volume.

As illustrated in FIG. 4, in order to ensure the stimulation intensity as well as limit the temperature rise (Temperature rise less than 1°C in the brain is considered safe.), each electrode group 21 is preferred to have at least two or more electrodes. In other embodiment, the lead 2 can include three or more electrode groups 21, to stimulate jointly more functional areas of the brain.

FIG. 5 schematically depicts implanted components of a DBS device. The implanted pulse generator 1 includes a control module (CPU), a power module electrically connected with the control module and a stimulation output module electrically connected with the control module and the power module. The first and the second contact groups 31 of the lead 2 electrically connect with the pulse generator 1 via conductive lines 3. Each contact independently connects with the pulse generator 1 via a conductive line 3. The stimulation output module of the pulse generator 1 is capable of outputting parameters, such as pulse width, amplitude and frequency, according to different requirements. As a result, the pulse generator 1 can accurately control each and every contact, and can deliver different stimulation parameters to different functional areas in the brain so as to improve therapeutic effect and safety.

In an embodiment, the first distance P1 is no less than 0.5mm.

In an embodiment, the second distance P2 is larger than 3mm.

In an embodiment, lengths of the electrodes 810, 820, 930, 940 are L1, L2, L3 and L4 in turn. All these lengths L1 to L4 are no less than 0.5mm. The lengths of the electrodes 810, 820, 930, 940 could be equal or different.

Three embodiments will be given hereinafter.

### First embodiment

P1=0.5mm
P2=3.5mm
P3=0.5mm
L1=0.5mm
L2=0.5mm
L3=0.5mm
L4=0.5mm

### Second embodiment

P1=2mm
P2=4mm
P3=4mm
L1=3mm
L2=3mm
L3=3mm
L4=3mm

### Third embodiment

A representative case: male, 38-yrs old, married; height: 180cm; weight: 60kg. This patient has used heroin for 16 years with 0.5g/day. He felt euphoria when using drugs, but suffered from withdrawal symptoms including irritability, streaming eyes, yawning, muscular soreness and sleeplessness when stopping the drugs. This patient has a strong craving for the drug. The patient has been treated for drug-addiction about ten times. The longest withdrawal history was eighteen months when he worked in other localities, but he relapsed soon after he went back hometown. The longest withdrawal history in the hometown was six months. Before treated with the present invention, the patient had sexual life approximately once per two months and was evaluated as moderate depression.

The present disclosed lead of DBS device was positioned into the patient's brain. 10 days after the implantation, electrical stimulation was provided to the patient with parameters shown in Table 1. Changes in ECoG, local field potential in targeted nuclei, as well as MRI and PET-CT in addiction-relative functional areas were observed after the stimulation. Till writing of this patent application, the patient has no urge for the drug. Four random urine tests for morphine were negative. Three times when got back to the hometown, the patient can still stop craving for the drug. He gained weight of 7kg, reaching 67kg. The frequency for sex life rose to two or three times per week. His wife has been pregnant for two months after the patient was treated with the present disclosure. The evaluation index for the patient's motion, energy and life quality apparently rose after the treatment. No complication was observed during the treatment. By-effects of the electrical stimulation included anaesthesia, mild dizziness, gentle excitement/depression, full/poor energy, which were disappeared after the optimization of the stimulation parameters. By now, the patient has abstained from drugs for 5 months.

**Table 1**

| *Functional areas* | *Amplitude (V)* | *Pulse width (µm)* | *Frequency (Hz)* |
|---|---|---|---|
| the nucleus accumbens | 2.20 | 240 | 145 |
| the anterior limb of internal capsule | 2.20 | 300 | 185 |

Referring to other cases, the psychological addiction to the drug apparently reduced after the treatment with the disclosure. Indexes for Hamilton Depression Scale (HAMD), symptom checklist (SCL-90), the short form health survey scale, the Obsessive Compulsive Scale (YALE-BROWN) and Wechsler Memory Scale (WMS) were obviously improved compared with pre-operation. There was no significant change in Eysenck Personality Questionnaire (EPQ) before and after the treatment. No complication related to the stereotactic surgery appears. The emotion, spirit, memory and life quality (especially the sexual life) of the patients were improved.

The present disclosure can accurately target at the nuclei, and simultaneously stimulate two neighboring functional areas. Meanwhile, the stimulation parameters of the present disclosure can be optimized to deliver appropriate stimulation to each electrode. By simultaneously stimulating the nucleus accumbens and the anterior limbs of the internal capsules, the present disclosure improves the effect and safety of DBS in the therapy of drug-addiction, OCD and depression, especially in abstain from drugs psychologically.

Moreover, the present disclosure is also capable of delivering pulse to other functional areas so as to disturb the activities of tissues or neurons. With one single lead implanted in the brain, it can cover more extensive functional areas by contrast with current technologies without implanting more leads. The present disclosure can also be applied to the therapy of other disorders relative to the brain, such as paralysis and Parkinson's disease as well as mental diseases. In one embodiment of the present disclosure, the first electrode group and the second electrode group simultaneously stimulate the nucleus accumben and the anterior limb of an internal capsule in the brain.

It is to be understood, however, that even though numerous characteristics and advantages of preferred and exemplary embodiments have been set out in the foregoing description, together with details of the structures and functions of the embodiments, the disclosure is illustrative only; and that changes may be made in detail within the principles of present disclosure to the full extent indicated by the broadest general meaning of the terms in which the appended claims are expressed.

## Claims

1. A device for deep brain stimulation comprising:
a control module outside of a brain;
a power module electrically connected with the control module;
a stimulation output module electrically connected with the control module and the power module; and
a lead adapted for being implanted in the brain and electrically connected with the stimulation output module, wherein the lead comprises: a proximal section and a distal section, wherein
the proximal section comprises a first contact group (830 and 840) and a second contact group (950 and 960), the first contact group comprises two first contacts (830 and 840) and the second contact group comprises two second contacts (950 and 960);
the distal section comprises a first electrode group (810 and 820) and a second electrode group (930 and 940), the first electrode group (810 and 820) comprises two first electrodes (810 and 820) which are placed in a nucleus accumben of a brain; the second electrode group (930 and 940) comprises two second electrodes (930 and 940) which are placed in an anterior limb of an internal capsule of the brain; and the first electrode group (810 and 820) and the second electrode group (930 and 940) are configured to stimulate the nucleus accumben and the anterior limb of the internal capsule in the brain simultaneously;
the two first contacts are electrically connected to the two first electrodes in a one-to-one correspondence, and the second two contacts are electrically connected to the second two electrodes in a one-to-one correspondence;
the first two contacts and the second two contacts are used for receiving stimulation signals from the control module and transmitting the stimulation signals to the two first electrodes and the two second electrodes for separate and accurate stimulation;
the stimulation parameters of the first electrode group and stimulation parameters of the second electrode group are independently adjustable; and
the stimulation frequency of the first electrode group (810 and 820) is lower than that of the second electrode group (930 and 940).

2. The device according to claim 1, wherein the two first electrodes (810 and 820, 930 and 940) are separated by a first distance P1, the first electrode group and the second electrode group are separated by a second distance P2, and the second distance P2 is larger than the first distance P1.

3. The device according to claim 2, wherein the second distance P2 is larger than 3 mm.

4. The device according to claim 2, wherein the first distance P1 is no less than 0.5 mm.

5. The device according to claim 1, wherein the lead is activated to cause the first electrode group and the second electrode group to simulate the nucleus accumben and anterior limb of an internal capsule in the brain simultaneously.

## Patentansprüche

1. Eine Vorrichtung zur Tiefenhirnstimulation, die Folgendes aufweist:
ein Steuermodul außerhalb eines Gehirns;
ein Leistungsmodul, das elektrisch mit dem Steuermodul verbunden ist;
ein Stimulationsausgangsmodul, das elektrisch mit dem Steuermodul und dem Leistungsmodul verbunden ist; und
eine Leitung, die dazu geeignet ist, in das Gehirn implantiert und elektrisch mit dem Stimulationsausgangsmodul verbunden zu sein, wobei die Leitung Folgendes aufweist: einen proximalen Abschnitt und einen distalen Abschnitt, wobei
der proximale Abschnitt eine erste Kontaktgruppe (830 und 840) und eine zweite Kontaktgruppe (950 und 960) aufweist, wobei die erste Kontaktgruppe zwei erste Kontakte (830 und 840) aufweist und wobei die zweite Kontaktgruppe zwei zweite Kontakte (950 und 960) aufweist;
der distale Abschnitt eine erste Elektrodengruppe (810 und 820) und eine zweite Elektrodengruppe (930 und 940) aufweist, wobei die erste Elektrodengruppe (810 und 820) zwei erste Elektroden (810 und 820) aufweist, die in einem Nucleus acumben eines Gehirns angeordnet sind; wobei die zweite Elektrodengruppe (930 und 940) zwei zweite Elektroden (930 und 940) aufweist, die in einem vorderen Glied einer inneren Kapsel des Gehirns platziert sind; und wobei die erste Elektrodengruppe (810 und 820) und die zweite Elektrodengruppe (930 und 940) eingerichtet sind, den Nucleus accumben und das vordere Glied der inneren Kapsel im Gehirn gleichzeitig stimulieren;
die beiden ersten Kontakte elektrisch mit den beiden ersten Elektroden in einer Eins-zu-eins-Entsprechung verbunden sind und wobei die beiden zweiten Kontakte elektrisch mit den beiden zweiten Elektroden in einer Eins-zu-eins-Entsprechung verbunden sind;
die beiden ersten Kontakte und die beiden zweiten Kontakte zum Empfangen von Stimulationssignalen vom Steuermodul und zum Übertragen der Stimulationssignale an die beiden ersten Elektroden und die beiden zweiten Elektroden für eine getrennte und genaue Stimulation verwendet werden;
die Stimulationsparameter der ersten Elektrodengruppe und die Stimulationsparameter der zweiten Elektrodengruppe unabhängig voneinander einstellbar sind; und
die Stimulationsfrequenz der ersten Elektrodengruppe (810 und 820) niedriger ist als die der zweiten Elektrodengruppe (930 und 940).

2. Die Vorrichtung nach Anspruch 1, wobei die beiden ersten Elektroden (810 und 820, 930 und 940) durch einen ersten Abstand P1 getrennt sind, wobei die erste Elektrodengruppe und die zweite Elektrodengruppe durch einen zweiten Abstand P2 getrennt sind und wobei der zweite Abstand P2 größer als der erste Abstand P1 ist.

3. Die Vorrichtung nach Anspruch 2, wobei der zweite Abstand P2 größer als 3 mm ist.

4. Die Vorrichtung nach Anspruch 2, wobei der erste Abstand P1 nicht kleiner als 0,5 mm ist.

5. Die Vorrichtung nach Anspruch 1, wobei die Leitung aktiviert wird, um zu veranlassen, dass die erste Elektrodengruppe und die zweite Elektrodengruppe den Nucleus accumben und das vordere Glied einer inneren Kapsel im Gehirn gleichzeitig stimulieren.

## Revendications

1. Dispositif pour stimulation cérébrale profonde, comprenant :
un module de commande externe à un cerveau ;
un module d'alimentation connecté électriquement au module de commande ;
un module de sortie de stimulation connecté électriquement au module de commande et au module d'alimentation ; et
un conducteur adapté pour être implanté dans le cerveau et connecté électriquement au module de sortie de stimulation, dans lequel le conducteur comprend : une section proximale et une section distale, dans lequel
la section proximale comprend un premier groupe de contacts (830 et 840) et un deuxième groupe de contacts (950 et 960), le premier groupe de contacts comprend deux premiers contacts (830 et 840) et le deuxième groupe de contacts comprend deux deuxièmes contacts (950 et 960) ;
la section distale comprend un premier groupe d'électrodes (810 et 820) et un deuxième groupe d'électrodes (930 et 940), le premier groupe d'électrodes (810 et 820) comprend deux premières électrodes (810 et 820) qui sont placées dans un noyau accumbens d'un cerveau ; le deuxième groupe d'électrodes (930 et 940) comprend deux deuxièmes électrodes (930 et 940) qui sont placées dans un bras antérieur d'une capsule interne du cerveau ; et le premier groupe d'électrodes (810 et 820) et le deuxième groupe d'électrodes (930 et 940) sont configurés pour stimuler simultanément le noyau accumbens et le bras antérieur de la capsule interne dans le cerveau ;
les deux premiers contacts sont connectés électriquement aux deux premières électrodes selon une correspondance biunivoque, et les deux deuxièmes contacts sont connectés électriquement aux deux deuxièmes électrodes selon une correspondance biunivoque ;
les deux premiers contacts et les deux deuxièmes contacts sont utilisés pour recevoir des signaux de stimulation provenant du module de commande et pour émettre les signaux de stimulation aux deux premières électrodes et aux deux deuxièmes électrodes pour une stimulation distincte et précise ;
les paramètres de stimulation du premier groupe d'électrodes et les paramètres de stimulation du deuxième groupe d'électrodes sont réglables indépendamment ; et
la fréquence de stimulation du premier groupe d'électrodes (810 et 820) est inférieure à celle du deuxième groupe d'électrodes (930 et 940).

2. Dispositif selon la revendication 1, dans lequel les deux premières électrodes (810 et 820, 930 et 940) sont séparées par une première distance P1, le premier groupe d'électrodes et le deuxième groupe d'électrodes sont séparés par une deuxième distance P2, et la deuxième distance P2 est supérieure à la première distance P1.

3. Dispositif selon la revendication 2, dans lequel la deuxième distance P2 est supérieure à 3 mm.

4. Dispositif selon la revendication 2, dans lequel la première distance P1 n'est pas inférieure à 0,5 mm.

5. Dispositif selon la revendication 1, dans lequel le conducteur est activé pour amener le premier groupe d'électrodes et le deuxième groupe d'électrodes à stimuler simultanément le noyau accumbens et le bras antérieur d'une capsule interne dans le cerveau.
